Europäisches Patentamt

European Patent Office

Office européen des brevets

(19)

(11) Veröffentlichungsnummer: **0 276 474 B1**

(12) **EUROPÄISCHE PATENTSCHRIFT**

(45) Veröffentlichungstag der Patentschrift: 02.05.91

(51) Int. Cl.⁵: **A61M 5/315**

(21) Anmeldenummer: 87119152.4

(22) Anmeldetag: 23.12.87

(54) **Dosiervorrichtung.**

(30) Priorität: 30.01.87 DE 8701486 U

(43) Veröffentlichungstag der Anmeldung:
03.08.88 Patentblatt 88/31

(45) Bekanntmachung des Hinweises auf die
Patenterteilung:
02.05.91 Patentblatt 91/18

(84) Benannte Vertragsstaaten:
AT BE CH DE FR GB IT LI NL

(56) Entgegenhaltungen:
EP-A- 0 109 913
US-A- 80 793
US-A- 3 517 668

(73) Patentinhaber: THERA Patent GmbH & Co. KG
Gesellschaft für industrielle Schutzrechte
Griesberg 2
W-8031 Seefeld 1(DE)

(72) Erfinder: Herold, Wolf-Dietrich
Hoehenweg 13
W-8031 Seefeld 2(DE)

(74) Vertreter: Strehl, Schübel-Hopf, Groening
Maximilianstrasse 54 Postfach 22 14 55
W-8000 München 22(DE)

## Beschreibung

Dosiervorrichtung

Eine Dosiervorrichtung gemäß dem Oberbegriff des Anspruchs 1 ist aus EP-A1-O 080 793 und ähnlich auch aus EP-A20 109 913 und US-A-3 517 668 bekannt. Bei allen diesen Vorrichtungen nach dem Stand der Technik wird ein die auszubringende Substanz enthaltender Behälter in eine zweiteilige Aufnahme eingelegt, wobei die beiden Teile anschließend miteinander verschraubt oder in sonstiger Weise zusammengefügt werden. Im einen Teil der Aufnahme ist eine Kolbenstange mit über ihre Länge vorgesehenen Rastvorsprüngen angeordnet, in die eine Schaltklinke eingreift. Die Schaltklinke ist in einem Betätigungshebel schwenkbar gelagert, der seinerseits an dem die Kolbenstange enthaltenden Teil der Aufnahme schwenkbar gelagert ist. Eine zwischen der Schaltklinke und dem Betätigungshebel angeordnete Rückstellfeder spannt die Schaltklinke in Eingriff mit der Rastung der Kolbenstange und den Betätigungshebel in seine Ruhestellung vor.

Sämtliche bekannten Vorrichtungen stellen wegen der Vielzahl der genannten einzelnen Bauteile ein verhältnismäßig schweres, kompliziertes und in der Herstellung teures Gerät dar. Der die jeweilige Substanz enthaltende Behälter ist in die Vorrichtung einzulegen und nach Entleerung gegen einen neuen Behälter auszutauschen, wobei die zweiteilige Aufnahme zerlegt und wieder zusammengesetzt werden muß. Dadurch wird die Handhabung kompliziert. Die Vielzahl der Einzelteile und die Tatsache, daß die bekannten Geräte massiv und gewöhnlich aus Metall hergestellt sind, stehen ferner einer feinfühligen Handhabung im Wege, wie sie insbesondere für die Anwendung im medizinischen und zahnmedizinischen Bereich von Bedeutung ist.

Der Erfindung liegt die Aufgabe zugrunde, eine gattungsgemäße Vorrichtung anzugeben, die im Aufbau einfach, in der Fertigung unaufwendig, einfach zu handhaben und leicht ist.

Die erfindungsgemäße Lösung dieser Aufgabe ist im Kennzeichenteil des Anspruchs 1 angegeben. Die danach vorgesehene Gestaltung der den Behälter mit der Substanz lediglich an dessen hinterem Ende erfassenden und daher entsprechend kleinen Fassung mit sämtlichen für den Vorschub der Kolbenstange durch Einhand-Bedienung erforderlichen Funktionsteilen als einstükkiges Formteil, das gemäß Anspruch 3 vorzugsweise ein Kunststoff-Spritzteil sein kann, führt nicht nur zu der gewünschten kostengünstigen Fertigung und dem vorteilhaften geringen Gewicht, sondern auch dazu, daß diese Vorrichtung als Einwegteil eingesetzt und mit dem entleerten Behälter weggeworfen werden kann. Dabei kann der Behälter mit der Dosiervorrichtung bereits beim Abfüller zusammengebaut werden, so daß dem Benutzer ein sofort betriebsbereites Gerät zur Verfügung steht.

In der Ausgestaltung nach Anspruch 2 ist ferner auch eine Halteraste, die beim Stand der Technik als separate Klinke an der Aufnahme gelagert und durch eine eigene Feder gegen die Rastung der Kolbenstange vorgespannt ist, in das einstückige Formteil integriert.

Die Weiterbildung der Erfindung nach Anspruch 4 ist insofern von Vorteil, als bei dieser Gestaltung trotz Anformung der Schaltraste an den Betätigungshebel ein ausreichender Hub für den Vorschub der Kolbenstange beim Drücken des Betätigungshebels erzielt wird.

In der Weiterbildung der Erfindung nach Anspruch 5 ergibt sich der Vorteil, daß die Feder am längstmöglichen Hebelarm angreift, daher verhältnismäßig schwach sein kann und auch bei Fertigung aus Kunststoff ohne weiteres die erforderliche Rückstellkraft erzeugen kann. Gleichzeitig wird, ähnlich wie in der Weiterbildung nach Anspruch 8, eine nach außen hin weitgehend geschlossene Form der Vorrichtung erreicht.

Die Ausgestaltung der Erfindung nach den Ansprüchen 5 und 7 sind unter dem Gesichtspunkt der für ein einstückiges Formteil bedeutsamen einfachen Formgebung günstig. Das Merkmal des Anspruchs 9 stellt eine herstellungstechnisch einfache Maßnahme zur Erzielung der gewünschten Beweglichkeit des Betätigungshebels dar, während sich Anspruch 10 auf eine zweckmäßige Maßnahme zur Erzielung der erforderlichen Steifigkeit des Winkelhebels bezieht.

Die Weiterbildung der Erfindung nach Anspruch 11 ergibt die Möglichkeit einer unaufwendigen und im Betrieb unlösbaren Verbindung zwischen der Vorschubeinrichtung und dem die Substanz enthaltenden Behälter, wobei die Druckbetätigung auch bei ungenauen Fertigungstoleranzen nicht zu einem Verklemmen Kolbenstange in der Fassung führen kann.

Ein bevorzugtes Ausführungsbeispiel der Erfindung wird nachstehend anhand der Zeichnung näher erläutert, in der

Fig. 1 einen Längsschnitt durch ein aus Behälter, Kolben, Kolbenstange und Dosiervorrichtung zusammengebautes Gerät zeigt, während

Fig. 2 eine Draufsicht auf die Dosiervorrichtung in Richtung des Pfeiles A nach Fig. 1 wiedergibt.

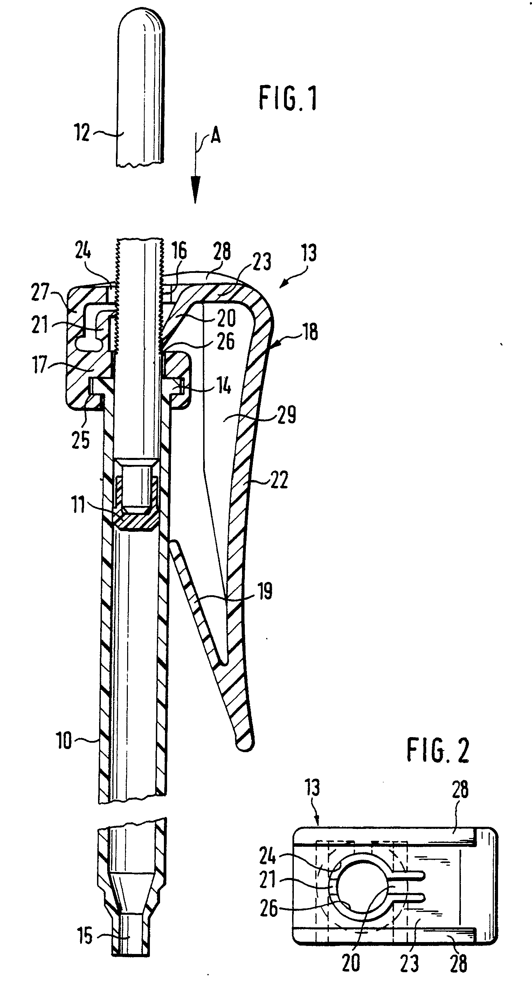

Das in Fig. 1 gezeigte Gerät besteht aus einem zylindrischen Behälter 10, einem in diesem gleitend verschiebbaren Kolben 11, einer Kolbenstange 12 und einer Dosiervorrichtung 13. Sämtliche Teile sind als Kunststoff-Spritzteile geformt.

Der Behälter 10 weist an seinem hinteren Ende einen Flansch 14 auf. An seinem vorderen Ausbringende 15 kann eine (nicht gezeigte) Injektionsnadel, Kanüle oder sonstige Ausbringvorrichtung aufgesteckt oder aufgeschraubt werden.

Der Raum zwischen dem Ausbringende 15 und dem in dem Behälter 10 verschiebbaren Kolben 11 dient zur Aufnahme der auszubringenden Substanz, bei der es sich beispielsweise um ein in der Zahnmedizin angewandtes Ätzgel oder ein sonstiges fließfähiges Material handeln kann. Die Kolbenstange 12 greift mit ihrem vorderen Ende in den Kolben 11 ein und dient dazu, diesen zum Ausbringen der Substanz vorzuschieben. Die Kolbenstange 12 ist über den Hauptteil ihrer Länge mit einer Rastung 16 versehen, bei der es sich um einzelne ringförmige Vorsprünge oder auch um ein Gewinde handeln kann.

Die als einstückiges Kunststoff-Formteil ausgebildete Dosiervorrichtung 13 weist eine Fassung 17, einen Betätigungshebel 18, eine blattförmige Rückstellfeder 19, eine Schaltraste 20 und eine Halteraste 21 auf. Der Betätigungshebel 18 ist im wesentlichen als Winkelhebel ausgebildet, dessen einer Arm 22 in der in Fig. 1 gezeigten Ruhestellung im wesentlichen parallel zu Behälter 10 und Kolbenstange 12 verläuft und dessen anderer Arm 23 sich senkrecht dazu erstreckt und eine Öffnung 24 zum Durchtritt der Kolbenstange 12 aufweist.

Die Fassung 17 ist mit einer Schiebeführung 25 versehen, mit der sie sich in der zur Zeichenebene der Fig. 1 senkrechten Richtung auf den Flansch 14 des Behälters 10 aufschieben läßt. Ferner ist in der Fassung 17 eine Öffnung 26 zum Durchtritt der Kolbenstange 12 vorgesehen.

Der Betätigungshebel 18 ist über ein an dem in Fig. 1 linken Ende des Arms 23 rechtwinklig ansetzendes Zwischenglied 27 mit der Fassung 17 verbunden, wobei der Materialquerschnitt an der Verbindungsstelle reduziert ist, so daß der Betätigungshebel 18 gegenüber der Fassung 17 leicht verschwenkbar ist. An der Außenseite des Arms 23 sind zwei auch in Fig. 2 sichtbare Rippen 28 angeformt. Eine weitere Rippe 29 ist an der Innenseite des Arms 22 angeformt, die sich über dessen gesamte Länge und über den angrenzenden Teil des Arms 23 erstreckt. Die Rippen 28 und 29 dienen dazu, den gewinkelten Betätigungshebel 28 zu versteifen.

Die Schaltraste 20 ist an dem Arm 23 des Betätigungshebels 18 im Bereich der Öffnung 24 angeformt, erstreckt sich unter einem spitzen Winkel zu diesem Arm 23 und liegt unter Spannung an der Kolbenstange 12 an, wobei am inneren freien Ende der Schaltraste 20 ausgebildete Zähne in die Rastung 16 der Kolbenstange 12 eingreifen.

Innerhalb der Verbindungsstelle des Zwischenglieds 27 mit der Fassung 17 ist die Halteraste 21 angeformt, wobei ebenfalls ein reduzierter Materialquerschnitt deren Beweglichkeit erhöht. Auch die Halteraste 21 weist an ihrem freien Ende, mit dem sie federnd in die Rastung 16 der Kolbenstange 12 eingreift, einige Zähne auf. Die Halteraste 21 befindet sich, wie aus Fig. 1 hervorgeht, innerhalb desvon der Fassung 17 und dem Betätigungshebel 18 mit seinem Zwischenglied 27 umgebenen Raum.

Die blattartige Rückstellfeder 19 ist am freien Ende des Arms 22 des Betätigungshebels 18 angeformt und verläuft unter einem spitzen Winkel zu diesem gemäß Fig. 1 nach oben und stützt sich mit ihrem freien Ende an der Außenwand des Behälters 10 ab. Auch die Rückstellfeder 19 liegt somit innerhalb des Betätigungshebels 18, wodurch eine insgesamt geschlossene Form der Dosiervorrichtung 13 erreicht wird.

Bei der Montage des in Fig. 1 gezeigten Geräts wird nach Befüllen des Behälters 10 mit der auszubringenden Substanz der Kolben 11 eingeführt. Sodann wird die Dosiervorrichtung 13 mit der Schiebeführung 25 in der zur Zeichenebene der Fig. 1 senkrechten Richtung auf den Flansch 14 aufgeschoben. Anschließend wird die Kolbenstange 12 durch die Öffnung 24 in dem Arm 23 des Betätigungshebels 18 und die Öffnung 26 in der Fassung 17 hindurchgeführt und in Anschlag bzw. (gemäß Fig. 1) in Eingriff mit dem Kolben 11 gebracht. Die Rastung 16 der Kolbenstange 12 und die an Schaltraste 20 und Halteraste 21 vorhandenen Zähne sind so gestaltet, daß eine Verschiebung der Kolbenstange 12 in dieser Richtung möglich ist.

Bei der Benutzung drückt die Bedienungsperson den Arm 22 des Betätigungshebels 18 in Richtung des Behälters 10, wobei dieser um die Verbindungsstelle zwischen dem Zwischenglied 27 und der Fassung 17 gemäß Fig. 1 im Uhrzeigersinn verschwenkt wird. Dabei führt die Schaltraste 22 gemäß Fig. 1 eine Abwärtsbewegung aus und verschiebt aufgrund ihres Eingriffs in die Rastung 16 die Kolbenstange 12 nach unten. Dadurch wird der Kolben 11 vorgeschoben und die im Behälter 10 enthaltene Substanz ausgebracht. Beim Loslassen des Betätigungshebels 18 wird dieser durch die Rückstellfeder 19 gemäß Fig. 1 gegen den Uhrzeigersinn in seine Ruhelage zurückverschwenkt, wobei die am freien Ende der Schaltraste 20 vorgesehenen Zähne an der Rastung 16 der Kolbenstange 12 nach oben gleiten. Eine Mitnahme der Kolbenstange in Aufwärtsrichtung wird dabei durch die Halteraste 21 verhindert. Bei erneutem Niederdrükken des Betätigungshebels 18 wird die Kolbenstange erneut nach unten vorgeschoben.

Ist der Kolben 11 in seiner vordersten Stellung am Ausbringende 15 angekommen und der Behälter 10 entleert, so wird er samt der Kolbenstange 12 und der Dosiervorrichtung 13 weggeworfen. Dies ist deshalb vertretbar, weil sämtliche Teile des Gerätes als billige Kunststoff-Spritzteile hergestellt sind.

**Ansprüche**

1. Vorrichtung zum dosierten Vorschub einer mit einer Rastung (16) versehenen Kolbenstange (12) relativ zu einem zylindrischen Behälter (10) zum Ausbringen einer darin enthaltenen fließfähigen Substanz, umfassend
   eine bezüglich des Behälters (10) fixierbare Aufnahme (17),
   einen relativ zu der Aufnahme (17) verschwenkbaren Betätigungshebel (18),
   eine den Betätigungshebel (18) in eine Ruhelage vorspannende Feder (19), und
   eine in die Rastung (16) der Kolbenstange (12) federnd eingreifende Schaltraste (20), die mit dem Betätigungshebel (18) verbunden ist und bei dessen Niederdrücken gegen die Federvorspannung die Kolbenstange (12) relativ zu der Aufnahme (17) verschiebt,
   dadurch gekennzeichnet, daß die Aufnahme (17) als eine nur am hinteren Ende des Behälters (10) angreifende Fassung ausgebildet ist, die mit dem Betätigungshebel (18), der Feder (19), und der Schaltraste (20) ein einstückiges Formteil bildet.

2. Vorrichtung nach Anspruch 1, gekennzeichnet durch eine ebenfalls an das Formteil angeformte Halteraste (21), die in die Rastung (16) der Kolbenstange (12) federnd eingreift und eine Rückbewegung der Kolbenstange (12) bei Rückkehr des Betätigungshebels (18) in seine Ruhelage verhindert.

3. Vorrichtung nach Anspruch 1 oder 2, dadurch gekennzeichnet, daß das Formteil ein Kunststoff-Spritzteil ist.

4. Vorrichtung nach einem der Ansprüche 1 bis 3, dadurch gekennzeichnet, daß der Betätigungshebel (18) als Winkelhebel ausgebildet ist, dessen einer Arm (23) eine Öffnung (24) zum Durchtritt der Kolbenstange (12) aufweist und an seinem Ende mit der Fassung (17) verbunden ist, und dessen anderer Arm (22) mit der Feder (19) versehen ist.

5. Vorrichtung nach Anspruch 4, dadurch gekennzeichnet, daß die Feder (19) am freien Ende des besagten anderen Arms (22) des Betätigungshebels (18) ansetzt und im spitzen Winkel zu diesem Arm (22) zur Anlage an einer Außenwand des Behälters (10) verläuft.

6. Vorrichtung nach Anspruch 4 oder 5, dadurch gekennzeichnet, daß die Schaltraste (20) an dem besagten einen Arm (23) auf der bezüglich dessen Verbindungsstelle mit der Fassung (17) gegenüberliegenden Seite der Öffnung (24) angeformt ist und im spitzen Winkel zu diesem verläuft.

7. Vorrichtung nach Anspruch 6, soweit dieser auf Anspruch 2 rückbezogen ist, dadurch gekennzeichnet, daß die Halteraste (21) auf der bezüglich der Schaltraste (20) gegenüberliegenden Seite der Öffnung (24) an der Fassung (17) angeformt ist.

8. Vorrichtung nach Anspruch 7, dadurch gekennzeichnet, daß der besagte eine Arm (23) des Betätigungshebels (18) über ein abgewinkeltes, zu dem anderen Arm (22) im wesentlichen parallel verlaufendes Zwischenglied (27) mit der Fassung (17) verbunden ist, und daß die Halteraste (21) innerhalb des Betätigungshebels (18) angeordnet ist.

9. Vorrichtung nach Anspruch 8, dadurch gekennzeichnet, daß das Material des Formteils an der Verbindungsstelle des zwischengliedes (27) mit der Fassung (17) eine Querschnittsverringerung aufweist.

10. Vorrichtung nach einem der Ansprüche 4 bis 9, dadurch gekennzeichnet, daß der Betätigungshebel (18) durch an der Außenseite des besagten einen Arms (23) und an der Innenseite es anderen Arms (22) angeformte Rippen (28, 29) versteift ist.

11. Vorrichtung nach einem der Ansprüche 1 bis 10, dadurch gekennzeichnet, daß die Fassung (17) zum Durchtritt der Kolbenstange (12) eine Öffnung (26) sowie zur Fixierung an einem am hinteren Ende des Behälters (10) vorgesehenen Flansch (14) eine Schiebeführung (25) aufweist, deren Achse quer zur Achse der Kolbenstange (12) und quer zur Wirkungsrichtung des auf den Betätigungshebel (18) auszuübenden Druckes verläuft.

**Claims**

1. A device for causing metered advancing of a piston rod (12) having catch means (16) relative to a cylindrical container (10) for dispensing a flowable substance contained therein, comprising
   receiving means (17) adapted to be fixed with respect to the container (10),
   an actuation lever (18) pivotable relative to the receiving means (17),
   a spring (19) biassing the actuation lever (18) to a rest position, and

an indexing detent (20) resiliently engaging the catch means (16) of the piston rod (12) and connected to the actuating lever (18) for advancing the piston rod (12) relative to the receiving means (17) upon depression of the lever,
characterised in that the receiving means (17) is formed as a socket engaging only the rear end of the container (10), the socket being formed with the actuation lever (18), the spring (19) and the indexing detent (20) in one integrally moulded part.

2. The device of claim 1, characterised by a retaining detent (21) also formed integrally with the moulded part and resiliently engaging the catch means (16) of the piston rod (12) to prevent a reverse motion of the piston rod (12) when the actuation lever (18) returns to its rest position.

3. The device of claim 1 or 2, characterised in that the moulded part is a synthetic resin injection moulded part.

4. The device of and one of claims 1 to 3, characterised in that the actuation lever (18) is formed as an angle lever, one arm (23) of which is provided with an opening (24) for passing the piston rod (12) therethrough and has its end connected to the socket (17), and the other arm (22) of which is provided with the spring (19).

5. The device of claim 4, characterised in that the spring (19) is connected to the free end of the said other arm (22) of the actuation lever (18) and extends at an acute angle relative to this arm (22) into contact with an outer wall of the container (10).

6. The device of claim 4 or 5, characterised in that the indexing detent (20) is formed on the said one arm (23) at the side of the opening (24) opposite the location where the one arm is connected to the socket (17), and extends at an acute angle relative to the one arm.

7. The device of claim 6 as dependent on claim 2, characterised in that the retaining detent (21) is formed on the socket (17) at the side of the opening (24) opposite the indexing detent (20).

8. The device of claim 7, characterised in that the said one arm (23) of the actuation lever (18) is connected to the socket (17) through a bent intermediate link (27) extending substantially parallel to the other arm (22), and that the retaining detent (21) is disposed inside the actuation lever (18).

9. The device of claim 8, characterised in that the material of the moulded part has a reduced cross-section at the location where the intermediate link (27) is connected to the socket (17).

10. The device of any one of claims 4 to 9, characterised in that the actuation lever (18) is stiffened by ribs (28, 29) formed on the outer side of the said one arm (23) and on the inner side of the other arm (22).

11. The device of any one of claims 1 to 10, characterised in that the socket (17) has an opening (26) for passing the piston rod (12) therethrough and a slide (25) for fixing the socket at a flange (14) provided at the rear end of the container (10), the axis of the slide extending transversely of the axis of the piston rod (12) and transversely of the direction of action of the pressure to be exerted on the actuation lever (18).

**Revendications**

1. Dispositif pour l'avancement dosé d'une tige de piston (12) munie d'une crémaillère (16) par rapport à un réservoir cylindrique (10) pour la distribution d'une substance fluide contenue à l'intérieur, comprenant un logement (17) pouvant être immobilisé par rapport au réservoir (10), un levier de manoeuvre (18) pouvant être pivoté par rapport au logement (17), un ressort (19) mettant le levier de manoeuvre (18) en précontrainte dans une position de repos, et un cliquet de commande (20) s'engageant élastiquement dans la crémaillère (16) de la tige de piston (12), qui est relié au levier de manoeuvre (18) et qui, lors de l'abaissement contre la précontrainte du ressort, déplace la tige de piston (12) par rapport au logement (17), **caractérisé en ce** que le logement (17) est réalisé sous la forme d'une douille fixée seulement sur l'extrémité arrière du réservoir (10) et constituant avec le levier de manoeuvre (18), le ressort (19) et le cliquet de commande (20) un élément profilé d'un seul tenant.

2. Dispositif selon la revendication 1, caractérisé en ce qu'il comprend un cran de maintien (21) également conformé sur l'élément profilé, qui s'engage élastiquement dans la crémaillère (16) de la tige de piston (12) et empêche un mouvement en arrière de ladite tige de piston (12) lors du retour du levier de manoeuvre (18) dans sa position de repos.

3. Dispositif selon l'une des revendications 1 ou 2, caractérisé en ce que l'élément profilé est une pièce en matière plastique moulée par injection.

4. Dispositif selon l'une des revendications 1 à 3, caractérisé en ce que le levier de manoeuvre (18) est conformé en levier coudé dont l'un des bras (23) comporte une ouverture (24) pour le passage de la tige de piston (12) et est rattaché à son extrémité à la douille (17), tandis que l'autre bras (22) est muni du ressort (19).

5. Dispositif selon la revendication 4, caractérisé en ce que le ressort (19) est fixé sur l'extrémité libre dudit autre bras (22) du levier de manoeuvre (18) et s'étend sous un angle aigu par rapport à ce bras (22) pour s'appliquer contre une paroi extérieure du réservoir (10).

6. Dispositif selon l'une des revendications 4 ou 5, caractérisé en ce que le cliquet de commande (20) est conformé sur le bras (23), du côté de l'ouverture (24) opposé au raccordement avec la douille (17), et forme avec celui-ci un angle aigu.

7. Dispositif selon la revendication 6, dans la mesure où celle-ci se rattache à la revendication 2, caractérisé en ce que le cran de maintien (21) est conformé sur la douille (17) du côté de l'ouverture (24) opposé au cliquet de commande (20).

8. Dispositif selon la revendication 7, caractérisé en ce que ledit bras (23) du levier de manoeuvre (18) est relié à la douille (17) par l'intermédiaire d'un élément intermédiaire (27) coudé et s'étendant sensiblement parallèlement à l'autre bras (22), et que le cran de maintien (21) est disposé à l'intérieur du levier de manoeuvre (18).

9. Dispositif selon la revendication 8, caractérisé en ce que la matière de l'élément profilé présente une diminution de la section au point de raccordement de l'élément intermédiaire (27) avec la douille (17).

10. Dispositif selon l'une des revendications 4 à 9, caractérisé en ce que le levier de manoeuvre (18) est renforcé par des nervures (28, 29) conformées sur la face extérieure dudit bras (23) et sur la face intérieure de l'autre bras (22).

11. Dispositif selon l'une des revendications 1 à 10, caractérisé en ce que la douille (17) comprend pour le passage de la tige de piston (12) une ouverture (26) ainsi que, pour la fixation sur une bride (14) prévue à l'extrémité arrière du réservoir (10), un guidage coulissant (25) dont l'axe s'étend transversalement à l'axe de la tige de piston (12) et transversalement à la direction d'action de la pression à exercer sur le levier de manoeuvre (18).

FIG.1

FIG. 2